# EUROPEAN PATENT SPECIFICATION

(11) **EP 1 355 681 B1**
(45) Date of publication and mention of the grant of the patent: **08.03.2006**
(21) Application number: 01994478.4
(22) Date of filing: 21.12.2001
(51) Int. Cl.: A61L 12/08, A61L 27/02, A61L 27/14, A45C 11/00, G02B 1/04, G02C 7/04

(54) **ANTIMICROBIAL CONTACT LENSES CONTAINING ACTIVATED SILVER AND METHODS FOR THEIR PRODUCTION**
AKTIVIERTES SILBER ENTHALTENDE ANTIMIKROBIELLE KONTAKTLINSE SOWIE VERFAHREN ZU DEREN HERSTELLUNG
LENTILLES DE CONTACT ANTIMICROBIENNES CONTENANT DE L'ARGENT ACTIF ET PROCEDE PERMETTANT DE PRODUIRE CES LENTILLES

(30) Priority: 21.12.2000 US 257317 P
(43) Date of publication of application: 29.10.2003
(73) Proprietor: Johnson & Johnson Vision Care, Inc., Jacksonville, FL 32256 (US)
(72) Inventor: VANDERLAAN, Douglas, G., Jacksonville, FL 32211 (US); MEYERS, Ann, Jacksonville, FL 32216 (US); BROWN-SKROBOT, Susan, Jacksonville, FL 32216 (US)
(74) Representative: Mercer, Christopher Paul
(86) International application number: PCT/US2001/050582
(87) International publication number: WO 2002/062402

(56) References cited:
- EP-A- 1 050 314
- WO-A-00/38552
- WO-A-98/18330
- US-A- 5 312 586
- US-A- 5 515 117
- NISSEN S ET AL: "[Antimicrobial efficacy of a silver layer on hydrogel lenses] Citation]" DER OPHTHALMOLOGE: ZEITSCHRIFT DER DEUTSCHEN OPHTHALMOLOGISCHEN GESELLSCHAFT. GERMANY SEP 2000, vol. 97, no. 9, September 2000 (2000-09), pages 640-643, XP002204043 ISSN: 0941-293X

## Description

### FIELD OF THE INVENTION

This invention relates to a method of producing optically clear lenses having antimicrobial properties as well as methods of their production, use, and storage.

### BACKGROUND OF THE INVENTION

Contact lenses have been used commercially to improve vision since the 1950s. The first contact lenses were made of hard materials. Although these lenses are currently used, they are not suitable for all patients due to their poor initial comfort and their relatively low permeability to oxygen. Later developments in the field gave rise to soft contact lenses, based upon hydrogels, which are extremely popular today. Many users find soft lenses are more comfortable, and increased comfort levels allow soft contact lens users to wear their lenses for far longer hours than users of hard contact lenses.

Despite this advantage, the extended use of the lenses can encourage the buildup of bacteria or other microbes, particularly, *Pseudomonas aeruginosa,* on the surfaces of soft contact lenses. The build-up of bacteria or other microbes is not unique to soft contact lens wearer and may occur during the use of hard contact lenses as well.

Therefore, there is a need to produce contact lenses which inhibit the growth of bacteria or other microbes and/or the adhesion of bacterial or other microbes on the surface of contact lenses. Further there is a need to produce contact lenses which do not promote the adhesion and/or growth of bacteria or other microbes on the surface of the contact lenses. Also there is a need to produce contact lenses which inhibit adverse responses related to the growth of bacteria or other microbes.

Others have recognized the need to produce soft contact lenses which inhibit the growth of bacteria. In US Patent No. 5,213,801, the production of an antibacterial contact lens is disdosed, where an antibacterial metal ceramic material within a soft contact lens is incorporated into a contact lens. This procedure contains a number of steps and may not be suitable for producing all types of lenses in a production environment. The steps include making a silver ceramic material that is fine enough to be used in a contact lens and then forming the lens with the powdered ceramic. However, lenses containing these types of materials often lack the clarity required by contact lens users.

Although these methods and lenses are known, other contact lenses that inhibit the growth and/or adhesion of bacteria or other microbes and are of sufficient optical clarity, as well as methods of making those lenses are still needed. It is this need, which this invention seeks to meet.

### DETAILED DESCRIPTION OF THE INVENTION

This invention includes a method of producing optically clear lens having antimicrobial properties comprising more than about 0.01 weight percent activated silver, based on the weight of the lens in the un-hydrated state and wherein said silver is incorporated into the polymer of the lens prior to forming the lens and is activated by treatment with an oxidizing agent. As used herein, the phrase "optically clear," refers to a lens that has optical clarity comparable to currently available commercial lenses, e.g. etafilcon A, balafilcon A, and the like. The term "lens" refers to opthalmic devices that reside in or on the eye. These devices can provide optical correction or may be cosmetic. The term lens includes but is not limited to soft contact lenses, hard contact lenses, intraocular lenses, overlay lenses, ocular inserts, and optical inserts. Typical hard contact lenses are made from polymers which include but are not limited to polymers of poly(methyl)methacrylate, silicone acrylates, fluoroacrylates, fluoroethers, polyacetylenes, and polyimides, where the preparation of representative examples may be found in JP 200010055, JP 6123860, and U.S. Patent 4,330,383. Typical soft contact lenses are made from silicone elastomers, or hydrogels, such as but not limited to silicone hydrogels and fluorohydrogels. The preparation of representative soft contact lenses may be found in US Patent No. 5,710,302, WO 9421698, EP 406161, JP 2000016905, U.S. Pat. No. 5,998,498, US Pat. App. No. 09/532,943,a continuation-in-part of US Pat App. No. 09/532,943, filed on August 30, 2000, and U.S. Patent No. 6,087,415. Examples of commercially available soft contact lenses include but are not limited to etafilcon A, genfilcon A, lenefilcon A, polymacon, and lotrafilcon A. The preferable contact lenses are etafilcon A, balafilcon A, and silicone hydrogels, as prepared in U.S. Pat. No. 5,998,498, US Pat. App. No. 09/532,943, a continuation-in-part of US Pat App. No. 09/532,943, filed on August 30, 2000, and U.S. Patent No. 6,087,415. Intraocular lenses of the invention can be formed using known materials. For example, the lenses may be made from a rigid material including, without limitation, polymethyl methacrylate, polystyrene, polycarbonate, or the like, and combinations thereof. Additionally, flexible materials may be used including, without limitation, hydrogels, silicone materials, acrylic materials, fluorocarbon materials and the like, or combinations thereof. Typical intraocular lenses are described in WO 0026698, WO 0022460, WO 9929750, WO 9927978, WO 0022459, and JP 2000107277. All of the aforementioned lenses of the invention, may be coated with a number of agents that are used to coat lens. For example, the procedures, compositions, and methods of U.S. Pat. No. 6,087,415 may be used and this patent is hereby incorporated by reference for those procedures, compositions, and methods.

The term, "activated silver," refers to silver that has been incorporated into the polymer of a lens, prior to forming the lens and subsequently activated by treatment with an oxidizing agent. Oxidizing agents include but are not limited to hydrogen peroxide, sodium hypochlorite, peroxy acids, bromine, chlorine, chromic acid, potassium permaganate, and iodine. The preferred oxidizing agent is sodium hypochlorite. The oxidizing agent can be dispersed or dissolved in an aqueous solution, such as deionized water, and the formed lens may be washed or soaked with this solution for a period of time. The concentration of the oxidizing agent in aqueous solution is about 0.1 to about 50 weight percent, where the percentage is based on the weight (or volume) of the solution, preferably about 0.4 to about, 30 weight percent, and more preferably about 0.6 to about 15 weight percent. The period of time for the treating the lenses with the oxidizing agent is about 10 seconds to about 10 hours, preferably about 1 to about 10 minutes.

The silver that is oxidized includes but is not limited to powdered silver having mesh size of -30, -60, or -325 or an average particle size of 2 to 8 microns; nanosize powder; and silver that is formed by reduction of ionic silver in the polymer matrix. The amount of silver in the lens is greater than 0.01 weight percent, where the percentage is based the weight of the components of the un-hydrated monomer. The weight percentage of silver is 0.01 to 0.3 weight percent, more preferably, 0.02 to 0.2 weight percent, and most preferably 0.03 to 0.1 weight percent.

The phrase, "antimicrobial properties," refers to a lenses that exhibit one or more of the following properties, the inhibition of the adhesion of bacteria or other microbes to the lenses, the inhibition of the growth of bacteria or other microbes on lenses, and the killing of bacteria or other microbes on the surface of lenses or in a radius extending from the lenses (hereinafter adhesion of bacteria or other microbes to lenses, the growth of bacteria or other microbes to lenses and the presence of bacterial or other microbes on the surface of lenses is collectively referred to as "microbial production"). Particularly, preferably, the lenses of the invention exhibit at least a 1-log reduction (≥ 90% inhibition) of viable bacteria or other microbes, most particularly preferably, about a 2-log reduction (≥ 99% inhibition) of viable bacteria or other microbes in in vitro tests. Such bacteria or other microbes include but are not limited to those organisms found in the eye, particularly *Pseudomonas aeruginosa, Acanthanmoeba, Staph. aureus, E. coli, Staphyloccus epidermidus,* and *Serratia marcesens.*

Thus a method of reducing a lens wearer's adverse microbial reactions comprises, consists essentially of, and consists of, the step of providing an optically dear lens having antimicrobial properties, the lens comprising, consisting essentially of, or consisting of more than about 0.01 weight percent activated silver. The terms lens, activated silver, optically clear, and antimicrobial properties all have their aforementioned meanings and preferred ranges. The preferred lens wearer is a human. The phrase "adverse events associated with microbial infections" include but are not limited to the following: ulcerative (microbial, infectious) keratitis, infiltrative keratitis, asymptomatic infiltrates, contact lens-induced peripheral ulcer, contact lens-induced acute red eye, and contact lens-induced papillary conjunctivitis. Although any reduction in the population of bacteria or other microbes in a lens wearer's eye may alleviate the adverse effects associated with microbial infections, it is preferred that the lenses of the invention inhibit the growth of bacteria and other microbes in standard in vitro tests at about 50% to about 100%, more preferably, about 80% to about 100%, most preferably, about 90% to about 100%. Particularly, preferably, the lenses of the invention exhibit at least a 1-log reduction (≥ 90% inhibition) of viable bacteria or other microbes, most particularly preferably, about a 2-log reduction (≥ 99% inhibition) of viable bacteria or other microbes

The invention also includes lenses produced by a method of producing an optically clear lens having antimicrobial properties, the lens comprising more than 0.01 weight percent activated silver based on the weight of the lens in the un-hydrated state, where the method comprises, consists essentially of, or consists of treating a lens containing silver with an oxidizing agent. The terms lens, activated silver, optically clear, and antimicrobial properties all have their aforementioned meanings and preferred ranges. The phrase "oxidizing agents" includes but is not limited to hydrogen peroxide, sodium hypochlorite, peroxy acids, bromine, chlorine, chromic acid, potassium permanganate and iodine, where the preferred oxidizing agent is sodium hypochlorite. Although the oxidizing agent can be applied to the lens in a number of ways, preferably it is dispersed or dissolved in an aqueous solution, such as deionized water, and the formed lens may be washed or soaked with this solution for a period of time. The concentration of the oxidizing agent in aqueous solution is about 0.1 to about 50 weight percent, preferably about 0.4 to about, 30 weight percent, and more preferably about 0.6 to about 15 weight percent, where the percentage is based on the weight of the solution. The period of time for the treating the lenses with the oxidizing agent is about 10 seconds to about 10 hours, preferably about 1 to 10 minutes.

In order to illustrate the invention the following examples are included.

### EXAMPLES

The following abbreviations are used in the examples below:
- HEMA: 2-hydroxyethyl methacrylate
- BAGE: glycerin esterified with boric acid
- EGDMA: ethyleneglycol dimethacrylate
- Darocur™ 1173: 2-hydroxy-2-methyl-1-phenyl-propan-1-one
- MAA: methacrylic acid
- TRIS: 3-methacryloxypropyltris (trimethylsiloxy) silane
- DMA: N,N-dimethylacrylamide
- THF: tetrahydrofuran
- TMI: dimethyl meta-isopropenyl benzyl isocyanate
- HEMA: 2-hydroxyethyl methacrylate
- TEGDMA: tetraethyleneglycol dimethacrylate
- MMA: methyl methacrylate
- TBACB: tetrabutyl ammonium-m-chiorobenzoate
- mPDMS: 800-1000 MW monomethacryloxypropyl terminated polydimethylsiloxane
- 3M3P: 3-methyl-3-pentanol
- Norbloc: 2-(2'-hydroxy-5-methacrylyloxyethylphenyl)-2H-benzotriazole
- CGI 1850: 1:1 (wgt) blend of 1-hydroxycyclohexyl phenyl ketone and bis(2,6-dimethoxybenzoyl)-2,4-4-trimethylpentyl phosphine oxide
- PVP: poly(N-vinyl pyrrolidone)
- IPA: isopropyl alcohol
- GMMA: glycerin 1-monomethacrylate
- mPEG 350: poly(ethylene glycol) methyl ether
- D30: 3,7-dimethyl-3-octanol
- TAA: t-amyl alcohol
- Blue HEMA: the reaction product of reactive blue number 4 and HEMA, as described in Example 4 or U.S. Pat. no. 5,944,853

### Biological Vortex Assay

The following viable bacteria adhesion assay was used where indicated in the following examples. A culture of *Pseudomonas aeruginosa,* ATCC# 15442 (ATCC, Rockville, MD) is grown overnight in a nutrient medium. The bacterial inoculum is prepared to result in a final concentration of 1 x 10⁸ colony forming units (cfu)/ml. Three contact lenses are rinsed with phosphate buttered saline ("PBS") pH 7.4 ± 0.2. Each washed contact lens is combined with two ml of the bacterial inoculum into a glass vial, which is agitated in a shaker-incubator for two hr. at 37 ±°C. Each lens is washed with PBS, placed into 10 ml of PBS containing 0.05% Tween™ 80 and vortexed at 2000 rpm for three min. The resulting supernatant is enumerated for viable bacteria, and the results of the detectacted viable bacteria attached to three lenses are averaged.

### Example 1

A blend of 9.80 g HEMA, 0.08 g EGDMA, 0.04 g Darocur™ 1173, and nanosize activated powdered silver (99.9+%, from Aldrich Chemicals), in 9.80 g of BAGE diluent, was sonicated for 1 hour. The resulting mixture was charged to a mold subsequently exposed to UV light for 30 minutes in a polystyrene to cure the polymer. After curing, the molds were opened, and the lenses were washed out into borate-buffered saline. The resulting lenses were soaked for 10 minutes in a 5.25% v/v solution of sodium hypochlorite in water, then rinsed 5 times in 0.85% v/v physiological saline solution. The number of viable *Pseudomonas aeruginosa* adhered to the lenses using the microbial assay described above was reduced by 99.8% compared to the untreated contact lens.

### Examples 2-4

The procedure of Example 1 was repeated, with addition of MAA as indicated in Table 1. In all cases the adhesion of viable bacteria was reduced when compared to a lens of the same polymer composition without silver. The results of the biological assay are shown in Table 1.

**Table 1**

| | Example 1 | Example 2 | Example 3 | Example 4 |
|---|---|---|---|---|
| | | | | |
| HEMA, g | 9.80 | 9.80 | 9.80 | 9.80 |
| MAA, g | 0.00 | 0.06 | 0.14 | 0.20 |
| EGDMA, g | 0.08 | 0.08 | 0.08 | 0.08 |
| Darocur 1173, g | 0.04 | 0.04 | 0.04 | 0.04 |
| Nanosize Ag | 0.022 | 0.022 | 0.022 | 0.022 |
| Reduction in viable bacteria* | 99.8% | 99.7% | 99.7% | 99.7% |

### Example 5

### Macromer B Preparation

To a dry container housed in a dry box under nitrogen at ambient temperature was added 30.0 g (0.277 mol) of bis(dimethylamino)methylsilane, a solution of 13.75 ml of a 1 M solution of TBACB (386.0 g TBACB in 1000 ml dry THF), 61.39 g (0.578 mol) of p-xylene, 154.28 g (1.541 mol) methyl methacrylate (1.4 equivalents relative to initiator), 1892.13 (9.352 mol) 2-(trimethylsiloxy)ethyl methacrylate (8.5 equivalents relative to initiator) and 4399.78 g (61.01 mol) of THF. To a dry, three-necked, round-bottomed flask equipped with a thermocouple and condenser, all connected to a nitrogen source, was charged the above mixture prepared in the dry box.

The reaction mixture was cooled, to 15°C while stirring and purging with nitrogen. After the solution reaches 15°C, 191.75 g (1.100 mol) of 1-trimethylsiloxy-1-methoxy-2-methylpropene (1 equivalent) was injected into the reaction vessel. The reaction was allowed to exotherm to approximately 62 °C and then 30 ml of a 0.40 M solution of 154.4 g TBACB in 11 ml of dry THF was metered in throughout the remainder of the reaction. After the temperature of reaction reached 30 °C and the metering began, a solution of 467.56 g (2.311 mol) 2-(trimethylsiloxy)ethyl methacrylate (2.1 equivalents relative to the initiator), 3636.6. g (3.463 mol) n-butyl monomethacryloxypropyl-polydimethylsiloxane (3.2 equivalents relative to the initiator), 3673.84 g (8.689 mol) TRIS (7.9 equivalents relative to the initiator) and 20.0 g bis(dimethylamino)methylsilane was added.

The mixture was allowed to exotherm to approximately 38-42 °C and then allowed to cool to 30 °C. At that time, a solution of 10.0 g (0.076 mol) bis(dimethylamino)methylsilane, 154.26 g (1.541 mol) methyl methacrylate (1.4 equivalents relative to the initiator) and 1892.13 g (9.352 mol) 2-trimethylsiloxy)ethyl methacrylate (8.5 equivalents relative to the initiator) was added and the mixture again allowed to exotherm to approximately 40 °C. The reaction temperature dropped to approximately 30 °C and 2 gallons of THF were added to decrease the viscosity. A solution of 439.69 g water, 740.6 g methanol and 8.8 g (0.068 mol) dichloroacetic acid was added and the mixture refluxed for 4.5 hours to de-block the protecting groups on the HEMA. Volatiles were then removed and toluene added to aid in removal of the water until a vapor temperature of 110°C was reached.

The reaction flask was maintained at approximately 110°C and a solution of 443 g (2.201 mol) TMI and 5.7 g (0.010 mol) dibutyltin dilaurate were added. The mixture was reacted until the isocyanate peak was gone by IR. The toluene was evaporated under reduced pressure to yield an off-white, anhydrous, waxy reactive monomer. The macromer was placed into acetone at a weight basis of approximately 2:1 acetone to macromer. After 24 hrs, water was added to precipitate out the macromer and the macromer was filtered and dried using a vacuum oven between 45 and 60 °C for 20-30 hrs.

### Lens Formation

A hydrogel was made from the following monomer mix (all amounts are calculated as weight percent of the total weight of the combination): macromer B (~18%), mPDMS (~28%), TRIS (~14%), DMA (~26%), HEMA (~5%), TEGDMA (~1%), PVP (~5%); CGI 1850 (~1%), glacial acetic acid (~5%), nanosize activated powdered silver (from Aldrich Chemicals~0.13%), with the balance comprising minor amounts of additives. The polymerization was conducted in the presence of 20%wt dimethyl-3-octanol diluent, and the blend was sonicated for 30 minutes before curing.

Contact lenses were formed by adding about 0.10 g of the monomer mix to the cavity of an eight-cavity lens mold of the type described in U.S. Patent 4,640,489. The lenses were cured for 8 minutes at 50 C (±5) using visible light (wavelength: 380-460 nm with a peak maximum at 425 nm, dose: approx. 2.5 J/cm²). After curing, the molds were opened, and the lenses were released into a 1:1 blend of water and ethanol, then leached in ethanol to remove any residual monomers and diluent. Finally the lenses were equilibrated in physiological borate-buffered saline. The lenses appeared transparent when examined with the naked eye, although the silver particles could be seen under magnification. The resulting lenses were soaked for 21 hours in a 5.25% solution of sodium hypochlorite in water, then rinsed 5 times in saline solution. The lenses were tested for antibacterial properties by the following method:

### Biological Broth Assay

Each lens was washed with Dulbecco's Phosphate Buffered Saline without calcium chloride and magnesium chloride, then placed into 1000 µl of Mueller Hinton Broth containing 10⁸ cfu/ml *Pseudomonas aeruginosa* (ATCC 15442), and incubated at 37°C overnight. The resulting solutions were observed for opacity and cultured to enumerate the bacteria, and compared to similar lenses that were not reacted with sodium hypochlorite. The results, in Table 3, show that the number of bacteria were reduced by >99.99%.

**Table 3**

| | Example 5 lens | Lenses without sodium hypochlorite |
|---|---|---|
| | | |
| Solution clarity | Clear | Opaque |
| Bacteria count cfu/ml | 3.8 x 10⁴ | 6.2 x 10⁸ |

### Example 6-8

A blend was made of 40.67 weight % HEMA, 1.0% Darocur 1173, 1.07% TEGDMA, 26.90% GMMA and 30.36% mPEG 350 (diluent). Nanosize activated powdered silver was added to this blend in amounts indicated in Table 4. Lenses were made and treated with sodium hypochlorite following the procedure of Example 1.

Lenses were autoclave sterilized, and tested for their antibacterial properties using the microbial assay described directly above. The results are shown in Table 4.

**Table 4**

| | Silver particles in blend | % Reduction in bacteria number* |
|---|---|---|
| Example 6 | 0.05 weight % | 99.7% |
| Example 7 | 0.10 weight % | 99.8% |
| Example 8 | 0.15 weight % | 99.6% |

| | | |
|---|---|---|
| *As compared to lenses of the same composition made without silver. | | |

### Examples 9-12 (not according to the invention)

Lenses were made using the blend and method described in Example 5, except without addition of powdered silver, and without reacting with sodium hypochlorite. These lenses were rinsed with deionized water to remove chloride ions. They were soaked in varying concentrations of AgNO₃, as indicated in Table 4 for 30 minutes, blotted to remove surface water, and placed into a solution of 5.0% ascorbic acid in water. After one hour the lenses were rinsed in deionized water, and then soaked either in 5.25% sodium hypochlorite or 3.0% aqueous hydrogen peroxide for 10 minutes or 60 minutes respectively, as indicated in Table 5. The lenses were rinsed in borate-buffered saline, autoclave sterilized, and tested for antibacterial properties using the vortex assay described above. The results are shown in Table 5

**Table 5**

| | [AgNO₃] | Oxidant | Oxidation time | % Reduction in bacteria number* |
|---|---|---|---|---|
| EXAMPLE 9 | 10⁻¹M | 5.25% NaOCl | 10 minutes | 99.9% |
| EXAMPLE 10 | 10⁻²M | 5.25% NaOCl | 10 minutes | 99.9% |
| EXAMPLE 11 | 10⁻³M | 5.25% NaOCI | 10 minutes | 99.9% |
| EXAMPLE 12 | 10⁻³M | 3.0% H₂O₂ | 60 minutes | 99.2% |

| | | | | |
|---|---|---|---|---|
| *As compared with a lens made without silver or oxidation. | | | | |

### EXAMPLES 13-16

The lenses of Example 5 were made, but with 0.10% nanosize silver, and instead of reacting with sodium hypochlorite, reacting with a solution of 3.0% hydrogen peroxide diluted to the indicated concentration with borate-buffered saline at room temperature for the time indicated in Table 6. The lenses were tested using the microbial assay described above. The results are shown in Table 6.

**Table 6**

| | Oxidant | Oxidation time | % Reduction in bacteria number* |
|---|---|---|---|
| | | | |
| EXAMPLE 13 | 1.5% H₂O₂ | 10 minutes | 96% |
| EXAMPLE 14 | 1.5% H₂O₂ | 30 minutes | 98% |
| EXAMPLE 15 | 1.5% H₂O₂ | 60 minutes | 98% |
| EXAMPLE 16 | 0.75% H₂O₂ | 60 minutes | 99% |

| | | | |
|---|---|---|---|
| *Compared to lenses of the same composition and are treated with H₂O₂, but made without silver. | | | |

### Examples 17-19

The lenses of Example 5 were made, but with 0.10% nanosize silver, and instead of reacting with sodium hypochlorite, reacting with a solution of 50% hydrogen peroxide diluted as needed to the indicated concentration with borate-buffered saline at room temperature for the time indicated in Table 7. The lenses were tested using the vortex assay described above. The results are shown in Table 7.

**Table 7**

| | Oxidant | Oxidation time | % Reduction in bacteria number* |
|---|---|---|---|
| | | | |
| EXAMPLE 17 | 3.0% H₂O₂ | 60 minutes | 96% |
| EXAMPLE 18 | 10.0% H₂O₂ | 60 minutes | 94% |
| EXAMPLE 19 | 50.0% H₂O₂ | 60 minutes | 91% |

| | | | |
|---|---|---|---|
| *Compared to lenses of the same composition and are treated with H₂O₂, but made without silver. | | | |

### EXAMPLES 20-22

The lenses of EXAMPLE 5 were made, but instead of reacting with sodium hypochlorite, reacting with an aqueous solution of 0.05M I₂ and 0.20 M KI at room temperature for the time indicated in Table 8. The lenses were tested using the vortex assay described above. The results are shown in Table 8.

**Table 8**

| | Oxidant | Oxidation time | % Reduction in bacteria number* |
|---|---|---|---|
| Example 20 | 0.05M I₂ | 15 minutes | 80% |
| Example 21 | 0.05M I₂ | 60 minutes | 90% |
| Example 22 | 0.05M I₂ | 1620 minutes | 92% |

| | | | |
|---|---|---|---|
| *Compared to an etafilcon A lens. The antibacterial activity of etatilcon A lenses is statistically the same as the activity of the lenses of example 5, when prepared without silver (95% confidence (p=0.09)). | | | |

### EXAMPLES 23-25

The lenses of EXAMPLE 5 were made, but with 0.20% nanosize silver, and instead of reacting with sodium hypochlorite, reacting with a solution of 3.0% hydrogen peroxide diluted to the indicated concentration with borate-buffered saline at room temperature for the time indicated in Table 9. The lenses were tested using the vortex assay described above. The results are shown in Table 9.

**Table 9**

| | Oxidant | Oxidation time | % Reduction in bacteria number* |
|---|---|---|---|
| | | | |
| EXAMPLE 23 | 1.5% H₂O₂ | 10 minutes | 93% |
| EXAMPLE 24 | 1.5% H₂O₂ | 60 minutes | 90% |
| EXAMPLE 25 | 1.5% H₂O₂ | 1440 minutes | 92% |

| | | | |
|---|---|---|---|
| *Compared to a similar H₂O₂-reacted lens made without silver. | | | |

### Examples 26-35

Nanosize silver can be added to the compositions listed in Table 10. Subsequently, lenses can be prepared as described in Example 5 and oxidized with 1-2 wgt.% H₂O₂. Macromers A and C were prepared as follows:

### Macromer A:

The procedure for Macromer B used except that 19.1 mole parts HEMA, 5.0 mole parts MAA, 2.8 mole parts MMA, 7.9 mole parts TRIS, 3.3 mole parts mPDMS, and 2.0 mole parts TMI were used.

### Macromer C:

The procedure for Macromer B was used except that 19.1 mole parts HEMA, 7.9 mole parts TRIS, 3.3 mole parts mPDMS, and 2.0 mole parts TMI were used.

**Table 10**

| EXAMPLE | 26 | 27 | 28 | 29 | 30 | 31 | 32 | 33 | 34 | 35 |
|---|---|---|---|---|---|---|---|---|---|---|
| Macromer | A | B | C | C | B | B | B | B | B | B |
| Macromer | 30.00 | 25.00 | 60.00 | 20.00 | 17.98 | 17.98 | 19.98 | 17.98 | 17.98 | 19.98 |
| TRIS | 0.00 | 18.00 | 0.00 | 40.00 | 21.00 | 21.00 | 8.00 | 20.00 | 25.00 | 20.00 |
| DMA | 27.00 | 28.00 | 36.00 | 36.00 | 25.50 | 25.50 | 26.00 | 22.00 | 9.00 | 23.00 |
| mPDMS | 39.00 | 18.00 | 0.00 | 0.00 | 21.00 | 21.00 | 28.50 | 25.50 | 30.00 | 28.50 |
| Norbloc | 2.00 | 2.00 | 3.00 | 3.00 | 2.00 | 2.00 | 2.00 | 2.00 | 2.00 | 2.00 |
| CGI 1850 | 2.00 | 1.00 | 1.00 | 1.00 | 1.00 | 1.00 | 1.00 | 1.00 | 1.00 | 1.00 |
| TEGDMA | 0.00 | 0.00 | 0.00 | 0.00 | 1.50 | 1.50 | 1.50 | 1.50 | 0.50 | 1.50 |
| HEMA | 0.00 | 0.00 | 0.00 | 0.00 | 5.00 | 5.00 | 5.00 | 5.00 | 7.00 | 5.00 |
| Blue HEMA | 0.00 | 0.00 | 0.00 | 0.00 | 0.02 | 0.02 | 0.02 | 0.02 | 0.02 | 0.02 |
| PVP | 0.00 | 8.00 | 0.00 | 0.00 | 5.00 | 5.00 | 8.00 | 5.00 | 7.50 | 9.00 |
| | | | | | | | | | | |
| Diluent % | 41 | 20 | 20 | None | 20 | 50.00 | 37.50 | 20.00 | 40.00 | 50.00 |
| Diluent | 3M3P | 3M3P | 3M3P | NA | D3O | TAA | 3M3P | TAA | 3M3P | 3M3P |

## Claims

1. A method of producing an optically clear lens having antimicrobial properties comprising more than about 0.01 weight percent activated silver, based on the weight of the lens in the un-hydrated state, and wherein said silver is incorporated into the polymer of the lens prior to forming the lens and is subsequently activated by treatment with an oxidizing agent.

2. The method of claim 1 wherein, the oxidizing agent is hydrogen peroxide, sodium hypochlorite, a peroxy acid, bromine, chlorine, chromic acid, potassium permanganate or iodine.

3. The method of claim 2 wherein the oxidizing agent is sodium hypochlorite.

4. An optically clear lens as prepared by the process of claim 1.

5. The lens of claim 4 wherein, the lens is a soft contact lens.

6. The lens of claim 4 wherein, the lens is a silicone hydrogel lens.

7. The lens of any one of claims 4 to 6 having 0.02 to 0.2 weight percent activated silver.

8. The lens of claim 7 having 0.05 to 0.2 weight percent activated silver.

9. The lens of claim 6 having 0.02 to 0.1 weight percent activated silver.

10. The lens of any one of claims 7 to 9 wherein the lens is lenefilcon A, aquafilcon A, etafilcon A, genfilcon A, balafilcon A, polymacon, or lotrafilcon A.

11. The lens of any one of claims 4 to 10 for use in reducing a lens wearer's adverse microbial reactions.

## Revendications

1. Procédé de production d'une lentille optiquement claire possédant des propriétés anti-microbiennes comprenant plus d'environ 0,01 pour cent en poids d'argent activé, sur la base du poids de la lentille dans un état non-hydraté, et dans lequel ledit argent est incorporé dans le polymère de la lentille avant la formation de la lentille et est activé par la suite par traitement avec un agent d'oxydation.

2. Procédé selon la revendication 1 dans lequel l'agent d'oxydation est le peroxyde d'hydrogène, l'hypochlorite de sodium, un peroxyacide, le brome, le chlore, l'acide chromique, le permanganate de potassium ou l'iode.

3. Procédé selon la revendication 2 dans lequel l'agent d'oxydation est l'hypochlorite de sodium.

4. Lentille optiquement claire qui est préparée selon le procédé de la revendication 1.

5. Lentille selon la revendication 4 dans laquelle la lentille est une lentille de contact souple.

6. Lentille selon la revendication 4 dans laquelle la lentille est une lentille silicone-hydrogel.

7. Lentille selon l'une quelconque des revendications 4 à 6 ayant entre 0,02 et 0,2 pour cent en poids d'argent activé.

8. Lentille selon la revendication 7 ayant entre 0,05 et 0,2 pour cent en poids d'argent activé.

9. Lentille selon la revendication 6 ayant entre 0,02 et 0,1 pour cent en poids d'argent activé.

10. Lentille selon l'une quelconque des revendications 7 à 9 dans laquelle la lentille est en lenefilcon A, en aquafilcon A, en etafilcon A, en genfilcon A, en balafilcon A, en polymacon, ou en lotrafilcon A.

11. Lentille selon l'une quelconque des revendications 4 à 10 pour son utilisation dans la réduction des réactions microbiennes indésirables chez un porteur de lentilles.

## Patentansprüche

1. Verfahren zur Herstellung einer optisch klaren Linse, die antimikrobielle Eigenschaften aufweist, umfassend mehr als ungefähr 0,01 Gewichtsprozent aktiviertes Silber, basierend auf dem Gewicht der Linse im nicht-hydrierten Zustand, und wobei das Silber in das Polymer der Linse inkorporiert wird, bevor die Linse geformt wird und anschließend durch Behandlung mit einem oxidierenden Mittel aktiviert wird.

2. Verfahren nach Anspruch 1, wobei das oxidierende Mittel Wasserstoffperoxid, Natriumhypochlorit, eine Peroxysäure, Brom, Chlor, Chromsäure, Kaliumpermanganat oder Jod ist.

3. Verfahren nach Anspruch 2, wobei das oxidierende Mittel Natriumhypochlorit ist.

4. Optisch klare Linse, wie durch das Verfahren nach Anspruch 1 hergestellt.

5. Linse nach Anspruch 4, wobei die Linse eine weiche Kontaktlinse ist.

6. Linse nach Anspruch 4, wobei die Linse eine Silikonhydrogel-Linse ist.

7. Linse nach einem der Ansprüche 4 bis 6, die von 0,02 bis 0,2 Gewichtsprozent aktiviertes Silber aufweist.

8. Linse nach Anspruch 7, die von 0,05 bis 0,2 Gewichtsprozent aktiviertes Silber aufweist.

9. Linse nach Anspruch 6, die von 0,02 bis 0,1 Gewichtsprozent aktiviertes Silber aufweist.

10. Linse nach einem der Ansprüche 7 bis 9, wobei die Linse Lenefilcon A, Aquafilcon A, Etafilcon A, Genfilcon A, Balafilcon A, Polymacon oder Lotrafilcon A ist.

11. Linse nach einem der Ansprüche 4 bis 10 zur Verwendung bei der Verringerung von adversen mikrobiellen Reaktionen des Trägers einer Linse.
